# EUROPÄISCHE PATENTANMELDUNG

(11) **EP 4 474 374 A1**
(43) Veröffentlichungstag der Anmeldung: **11.12.2024**
(21) Anmeldenummer: 23177898.6
(22) Anmeldetag: 07.06.2023
(51) Int. Cl.: C07C 29/151, C07C 31/04, B01J 8/00

(54) **METHANOLSYNTHESE MIT FLUKTUIERENDER ROHGASVERSORGUNG**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Peña, Vincent, 60439 Frankfurt am Main (DE); König, Sebastian, 60439 Frankfurt am Main (DE)
(74) Vertreter: Air Liquide

(57) **Zusammenfassung**

Anordnung (28) zur Methanolsynthese umfassend:
▪ eine Rohgasquelle (1),
▪ einen Zwischenspeicher (2),
▪ einen Verdichter (3),
▪ eine Synthesereaktoranordnung (4),

wobei die Rohgasquelle (1) über eine erste Hauptleitung (5) mit dem Verdichter (3) verbunden ist, wobei der Verdichter (3) über eine zweite Hauptleitung (6) mit der Synthesereaktoranordnung (4) verbunden ist,
wobei die zweite Hauptleitung (6) über eine erste Nebenleitung (7) mit einem Einlass (8) des Zwischenspeichers (2) verbunden ist,
wobei ein Auslass (9) des Zwischenspeichers (2) über eine zweite Nebenleitung (10) mit der zweiten Hauptleitung (6) verbunden ist und/oder über eine dritte Nebenleitung (11) mit der ersten Hauptleitung (5) verbunden ist.

## Beschreibung

Die vorliegende Erfindung betrifft eine Anordnung und ein Verfahren zur Methanolsynthese mit fluktuierender Rohgasversorgung.

Konventionelle Anlagen zur Methanolherstellung sind dahingehend ausgelegt, dass sie kontinuierlich bei einer konstanten Auslastung produzieren. Änderungen in der Auslastung sind im Normalbetrieb minimal. Dies wird durch die kontinuierliche Versorgung mit Synthesegasen ermöglicht. Lastwechsel und Prozessanpassungen zwischen definierten, stationären Zuständen erfolgen sehr langsam über Stunden bis Tage, um die Methanolanlage und den Katalysator zu schützen. Gewöhnliche Synthesegase aus Industrieprozessen enthalten hauptsächlich Wasserstoff, Kohlenstoffmonoxid und Kohlenstoffdioxid.

Die Herstellung von Methanol mit Wasserstoff, wobei der Wasserstoff mit Hilfe eines Elektrolyseurs aus erneuerbaren Energien produziert wird, ist vor die Herausforderung gestellt, dass die Wasserstoffproduktion mit Fluktuationen beaufschlagt ist. Die Fluktuationen der produzierten Wasserstoffmenge aus erneuerbaren Energien hängt beispielsweise von der verfügbaren Solarenergie und der verfügbaren Windenergie ab. Beispielsweise sinkt die Solarenergie in der Nacht ab oder die Windkraft sinkt während einer Sommerflaute. Typische Lastwechsel können sich hier innerhalb weniger Sekunden bis Minuten abspielen. Änderungen der Last des Elektrolyseurs sind in einer ähnlichen Größenordnung möglich. Lastwechsel bei fluktuierenden erneuerbaren Energien und des Elektrolyseurs sind jedoch um eine Größenordnung schneller als der Lastwechsel der Methanolanlage. Des Weiteren ist der zulässige Betriebsbereich der Methanolanlage stärker eingeschränkt als der des Elektrolyseurs und der erneuerbaren Energien.

Die Methanolsynthese verhält sich vergleichsweise träge. Damit ist sie der dominierende Engpass, da diese direkt mit dem Elektrolyseur verbunden ist.

Anlagen mit einem Zwischenspeicher zur Pufferung dieser Fluktuationen sind in der Literatur bekannt. Bei einer derartigen Anlage ist der Zwischenspeicher zwischen dem Elektrolyseur und der Methanolanlage platziert.

Der bekannte Stand der Technik hat allerdings den Nachteil, dass wenn kein Zwischenspeicher eingesetzt wird, einerseits bei einer Überkapazität von Wasserstoff der überschüssige Wasserstoff entsorgt werden muss. Dies kann schon dann vorliegen, sobald die Methanolanlage in Teillast betrieben wird und der Elektrolyseur spontan wieder mehr Wasserstoff zur Verfügung stellt. Anderseits muss bei einem rapiden Abfall der Wasserstoffmenge die Methanolanlage temporär mit konventionellem Wasserstoff aus fossilen Quellen betrieben werden oder die Methanolanlage muss für diese Fälle abgeschaltet werden.

Hiervon ausgehend liegt der vorliegenden Erfindung die Aufgabe zugrunde, einen Zwischenspeicher möglichst effizient in den Gesamtprozess einer Methanolsynthese einzugliedern und einen sicheren Betrieb der Methanolsynthese bei einer fluktuierenden Rohgasquelle zu ermöglichen.

Diese Aufgabe wird gelöst mit der Anordnung und dem Verfahren gemäß den unabhängigen Ansprüchen. Weitere vorteilhafte Ausgestaltungen der Erfindung sind in den abhängig formulierten Ansprüchen angegeben. Die in den abhängig formulierten Ansprüchen einzeln aufgeführten Merkmale sind in technologisch sinnvoller Weise miteinander kombinierbar und können weitere Ausgestaltungen der Erfindung definieren. Darüber hinaus werden die in den Ansprüchen angegebenen Merkmale in der Beschreibung näher präzisiert und erläutert, wobei weitere bevorzugte Ausgestaltungen der Erfindung dargestellt werden.

Erfindungsgemäß wird eine Anordnung zur Methanolsynthese vorgestellt, welche eine Rohgasquelle und einen Zwischenspeicher und einen ersten Verdichter und eine Synthesereaktoranordnung umfasst. Die Rohgasquelle ist über eine erste Hauptleitung mit dem ersten Verdichter verbunden. Der erste Verdichter ist über eine zweite Hauptleitung mit der Synthesereaktoranordnung verbunden. Die zweite Hauptleitung ist über eine erste Nebenleitung mit einem Einlass des Zwischenspeichers verbunden. Ein Auslass des Zwischenspeichers ist über eine zweite Nebenleitung mit der zweiten Hauptleitung und/oder über eine dritte Nebenleitung mit der ersten Hauptleitung verbunden.

Die Rohgasquelle ist dazu eingerichtet, eine oder mehrere Komponenten eines Rohgases bereitzustellen. Entsprechend ist die Rohgasquelle dazu eingerichtet, einen oder mehrere Rohgasströme bereitzustellen. Für die Funktionsweise der beschriebenen Anordnung kommt es nicht darauf an, ob das für die Methanolsynthese verwendete Rohgas aus einer einzelnen Rohgasquelle oder aus mehreren getrennten Rohgasquellen stammt.

Der Rohgasstrom beziehungsweise die Rohgasströme können fluktuieren. Fluktuierend kann sich dabei auf den Druck als auch auf den Massenstrom beziehen und meint die Änderung des Drucks oder des Massenstroms über die Zeit. Das Rohgas umfasst eines oder mehrere der Edukte der Methanolsynthese. Das Rohgas kann zumindest eines der Gasedukte Wasserstoff, Kohlenstoffdioxid und/oder Kohlenstoffmonoxid umfassen. Die einzelnen Edukte können als ein gemeinsamer Rohgasstrom bereitgestellt werden oder als einzelne Rohgasströme. Einzelne Rohgasströme können dabei unterschiedlich rein sein. Das Rohgas kann Begleitstoffe enthalten, die unter den Bedingungen der Methanolsynthese inert sind, wie beispielsweise Stickstoff oder Methan.

Zumindest einer der Rohgasströme stammt vorzugsweise aus einem Wasserelektrolyseprozess. Der Wasserelektrolyseprozess ist vorzugsweise mit einer Versorgung von erneuerbarer Energie betrieben, welche fluktuieren kann. Zumindest einer der Rohgasströme kann aus einem Kohlenstoffdioxidabscheideprozess oder einer Kohlenstoffdioxidleitung stammen. Der Rohgasdruck kann insbesondere zwischen 1 bar und 100 bar liegen. Der Rohgasdruck kann bei mindestens 1 bar, vorzugsweise bei mindestens 30 bar liegen.

Der Zwischenspeicher dient dazu, zumindest ein Rohgas zwischenzuspeichern. Dies kann je nach Bedarf erfolgen. Für die Funktionsweise der beschriebenen Anordnung ist es unerheblich, ob das im Zwischenspeicher zwischengespeicherte Rohgas alle Edukte der Methanol-Synthese umfasst oder nur eines davon. Enthält das Rohgas nicht alle Edukte, können die übrigen anderweitig der Synthesereaktoranordnung zugeführt werden. Für die Funktionsweise der beschriebenen Anordnung ist unerheblich, auf welche Weise dies erfolgt.

Der Zwischenspeicher kann aus einem oder mehreren Tanks und/oder einem Rohrsystem und/oder einer oder mehreren Kavernen bestehen. Der Zwischenspeicher kann einen maximalen Nettoverbrauch des Rohgases in der Methanolsynthese für eine Minute, bevorzugt für sechs Stunden, besonders bevorzugt für mehr als zwölf Stunden speichern. Der Druck im Zwischenspeicher liegt vorzugsweise in einem Bereich von 5 bar bis 700 bar. Bevorzugt beträgt der Druck 5 bar bis 100 bar, 20 bar bis 100 bar oder 80 bar bis 700 bar. Der Druck im Zwischenspeicher kann konstant gehalten werden, indem das Speichervolumen durch Hinzuschalten oder Abtrennen von Tanks, Rohrsystemen und/oder Kavernen angepasst wird. Bei ausschließlicher Versorgung der Methanolsynthese aus dem Zwischenspeicher kann der Druck im Zwischenspeicher unter den Normaldruck des Rohgases fallen. Die Temperatur des Zwischenspeichers liegt vorzugsweise zwischen -50°C und 100°C.

Die Synthesereaktoranordnung dient dazu, Methanol aus dem Rohgas zu synthetisieren. Eine Auslastung der Synthesereaktoranordnung, der sogenannte Lastbereich, liegt vorzugsweise zu jedem Zeitpunkt des Betriebs der Anordnung zwischen 10 % und 100 %, vorzugsweise zwischen 20% und 100%. Ein Lastbereich von 100% wird auch Volllast genannt. Ein Lastbereich unter 100% wird auch Teillast genannt. Die Synthesereaktoranordnung kann beispielsweise zum Betrieb mit einer minimalen Teillast von 20% ausgelegt sein. Der Druck in der Synthesereaktoranordnung liegt vorzugsweise zwischen 20 bar und 200 bar, vorzugsweise zwischen 30 bar und 100 bar. Die Betriebstemperatur der Synthesereaktoranordnung liegt vorzugsweise zwischen 150°C und 300°C, vorzugsweise zwischen 200° und 300°C.

Die Anordnung dient dazu, Methanol herzustellen und dabei den Zwischenspeicher möglichst effizient in den Gesamtprozess einzugliedern. Die Anordnung dient weiterhin dem zuverlässigen Betrieb einer Synthesereaktoranordnung mit einer fluktuierenden Rohgasversorgung. Dies wird durch die nachfolgend beschriebene Einbindung des Zwischenspeichers in die Anordnung erreicht.

Die Rohgasquelle ist über eine erste Hauptleitung mit dem ersten Verdichter verbunden und der erste Verdichter ist über eine zweite Hauptleitung mit der Synthesereaktoranordnung verbunden. Die Rohgasquelle ist also über den ersten Verdichter mit der Synthesereaktoranordnung verbunden. Dadurch kann das Rohgas der Synthesereaktoranordnung zugeführt werden. Der Abschnitt der Leitung zwischen der Rohgasquelle und dem ersten Verdichter wird als erste Hauptleitung bezeichnet und der Abschnitt der Leitung zwischen dem ersten Verdichter und der Synthesereaktoranordnung als zweite Hauptleitung. Die erste Hauptleitung und die zweite Hauptleitung können gemeinsam als eine Leitung realisiert sein, in welche der erste Verdichter integriert ist.

Die zweite Hauptleitung ist über eine erste Nebenleitung mit einem Einlass des Zwischenspeichers verbunden. Rohgas kann also an einer Stelle zwischen dem ersten Verdichter und der Synthesereaktoranordnung aus der Leitung zwischen der Rohgasquelle und der Synthesereaktoranordnung abgezweigt werden. Die Abzweigung stromab des ersten Verdichters hat den Vorteil, dass der erste Verdichter genutzt werden kann, um das Rohgas für die Einspeicherung im Zwischenspeicher zu verdichten. Der erste Verdichter dient also nicht nur dazu, das Rohgas bei der Zuführung zu der Synthesereaktoranordnung zu verdichten. Ein gesonderter Verdichter zum Zwecke der Einspeicherung ist also nicht erforderlich.

Ein Auslass des Zwischenspeichers ist über eine zweite Nebenleitung mit der zweiten Hauptleitung verbunden und/oder über eine dritte Nebenleitung mit der ersten Hauptleitung verbunden. Der "und"-Fall ist bevorzugt. Das im Zwischenspeicher gespeicherte Rohgas kann also stromab des ersten Verdichters und/oder stromauf des ersten Verdichters wieder in die Leitung zwischen der Rohgasquelle und der Synthesereaktoranordnung eingeleitet werden. Stromab des Verdichters, also in die zweite Hauptleitung, wird das Rohgas vorzugsweise dann aus dem Zwischenspeicher eingespeist, wenn der Druck im Zwischenspeicher größer als ein Grenzwert ist. Der Grenzwert ist vorzugsweise anhand des Betriebsdrucks der Synthesereaktoranordnung gewählt. Wenn der Druck im Zwischenspeicher größer als der Grenzwert ist, ist eine weitere Verdichtung nicht erforderlich. Stromauf des ersten Verdichters, also in die erste Hauptleitung, wird das Rohgas vorzugsweise dann aus dem Zwischenspeicher eingespeist, wenn der Druck im Zwischenspeicher kleiner als der Grenzwert ist. In dem Fall kann das aus dem Zwischenspeicher entnommene Rohgas mit dem ersten Verdichter weiter verdichtet werden.

Durch die beschriebene Ausgestaltung gibt es bei der beschriebenen Anordnung keine klare Unterteilung zwischen Quellen- und Speichersystem einerseits und Synthesesystem andererseits. Bei einer Lösung mit einer solchen Trennung, welche aus dem Stand der Technik bekannt ist, würde das Quellen- und Speichersystem einen konstanten Rohgasstrom an das Synthesesystem übergeben. Fluktuationen der Quelle würden innerhalb des Quellen- und Speichersystems durch einen Speicher ausgeglichen. Das Synthesesystem mit dem Verdichter würde den konstanten Rohgasstrom verarbeiten. Bei der beschriebenen Anordnung ist der Zwischenspeicher stromab des ersten Verdichters an die Leitung zwischen der Rohgasquelle und der Synthesereaktoranordnung, also an die zweite Hauptleitung, angebunden. Insbesondere dadurch wird eine Trennung zwischen Quellen- und Speichersystem und Synthesesystem durchbrochen. Dies ermöglicht die zuvor beschriebene Nutzung des Verdichters dessen, was bei der beschriebenen Anordnung einem Synthesesystem entspricht, für die Einspeicherung im Zwischenspeicher. Dieser Verdichter wird hierin als der erste Verdichter bezeichnet.

Das Rohgas kann je nach Bedarf in dem Zwischenspeicher eingespeichert werden und zu einem späteren Zeitpunkt wieder aus dem Zwischenspeicher über die zweite Hauptleitung oder über die erste Hauptleitung und die zweite Hauptleitung in die Synthesereaktoranordnung eingespeist werden. Dies kann über Druckregulierungseinrichtungen gesteuert werden, insbesondere über Ventile. Diese können auf vielfältige Weise angeordnet sein. Nachfolgend werden insoweit bevorzugte Ausgestaltungen beschrieben.

Die Anordnung weist vorzugsweise ein erstes Ventil zwischen der zweiten Hauptleitung und der ersten Nebenleitung auf. Das erste Ventil ist vorzugsweise als ein 3-Wege-Ventil ausgebildet, welches in die zweite Hauptleitung integriert ist und an welches die erste Nebenleitung als ein Abzweig angebunden ist.

Das erste Ventil kann einerseits ungewollte Druckausgleiche verhindern, anderseits können Totzeiten verringert werden. Totzeiten können entstehen, wenn das Rohgas die erste Nebenleitung zum Zwischenspeicher durchquert.

Die Anordnung weist vorzugsweise ein zweites Ventil zwischen dem Auslass des Zwischenspeichers und der zweiten Nebenleitung und/oder zwischen dem Auslass des Zwischenspeichers und der dritten Nebenleitung auf. Beispielsweise kann das zweite Ventil als ein 3-Wege-Ventil ausgebildet sein, wobei ein Einlass des zweiten Ventils mit dem Zwischenspeicher verbunden ist, wobei die zweite Nebenleitung an einem ersten Auslass des zweiten Ventils angebunden ist und die dritte Nebenleitung an einem zweiten Auslass des zweiten Ventils angebunden ist. In dem Fall sind die zweite Nebenleitung und die dritte Nebenleitung über eine gemeinsame Leitung zwischen dem zweiten Ventil an den Auslass des Zwischenspeichers angebunden.

Das zweite Ventil kann gezielt eine Verbindung zwischen dem Auslass des Zwischenspeichers und der ersten Hauptleitung über die dritte Nebenleitung oder zwischen dem Auslass des Zwischenspeichers und der zweiten Hauptleitung über die zweite Nebenleitung herstellen. Durch ein zweites Ventil können Totzeiten verringert werden. Totzeiten können entstehen, wenn das Rohgas vom Zwischenspeicher die zweite oder dritte Nebenleitung durchquert.

Die zweite Nebenleitung ist vorzugsweise mit der zweiten Hauptleitung stromaufwärts von der Verbindung der ersten Nebenleitung und der zweiten Hauptleitung verbunden.

Diese Anordnung ermöglicht ein rezirkulieren von Rohgas zum Zwischenspeicher, welches im vorliegenden Lastbereich nicht in der Synthesereaktoranordnung umgesetzt werden könnte.

Die wesentliche Funktionsweise der beschriebenen Anordnung lässt sich aber auch erzielen, wenn die zweite Nebenleitung mit der zweiten Hauptleitung stromab von der Verbindung der ersten Nebenleitung und der zweiten Hauptleitung verbunden ist.

In einer bevorzugten Ausführungsform ist die erste Hauptleitung über eine vierte Nebenleitung mit dem Einlass des Zwischenspeichers verbunden.

In dieser Ausführungsform kann Rohgas, welches im aktuellen Lastbereich nicht in der Synthesereaktoranordnung umgesetzt werden könnte, direkt aus der ersten Hauptleitung in den Zwischenspeicher eingespeichert werden.

Vorzugsweise verbindet ein drittes Ventil die vierte Nebenleitung mit der ersten Hauptleitung. Das dritte Ventil ist vorzugsweise als ein 3-Wege-Ventil ausgebildet, welches in die erste Hauptleitung integriert ist und an welches die vierte Nebenleitung als ein Abzweig angebunden ist. Das dritte Ventil kann Rohgas mit erhöhtem Druck zumindest teilweise schon vor dem ersten Verdichter abzweigen und weitere Verdichtungsarbeit im ersten Verdichter einsparen.

In einer weiteren bevorzugten Ausführungsform umfasst die Anordnung weiterhin einen zweiten Verdichter, welcher parallel zum ersten Verdichter geschaltet ist.

In dieser Ausführungsform kann der erste Verdichter zum Einspeichern von Rohgas verwendet werden. Insbesondere kann eine Komponente des Rohgases, bevorzugt Wasserstoff, mit dem ersten Verdichter eingespeichert werden. Eine weitere Komponente des Rohgases kann mit dem zweiten Verdichter auf einen geeigneten Betriebsdruck für die Synthesereaktoranordnung verdichtet werden. Ein paralleler zweiter Verdichter kann einen flexibleren Betrieb als auch den Betrieb mit unterschiedlichen Rohgasquellen ermöglichen.

In einer weiteren bevorzugten Ausführungsform ist ein dritter Verdichter dem Einlass des Zwischenspeichers vorgeschaltet.

In dieser Ausführungsform kann das Rohgas zusätzlich mit einem höheren Druck im Zwischenspeicher eingespeichert werden. Im Zwischenspeicher kann durch den höheren Druck mehr Rohgas eingespeichert werden.

In dieser Ausführungsform wird der oben beschriebene Vorteil nicht mehr erzielt, dass auf einen gesonderten Verdichter für die Einspeicherung im Zwischenspeicher verzichtet werden kann. Im Vergleich zu Lösungen aus dem Stand der Technik sind die Anforderungen an den dritten Verdichter der vorliegenden Ausführungsform allerdings geringer als an einen gesonderten Verdichter für ein vollständig vom Synthesesystem getrennten Quellen- und Speichersystem.

Der dritte Verdichter ist vorzugsweise parallel zu einem Leitungsabschnitt der ersten Nebenleitung geschaltet. Weist die Anordnung auch die vierte Nebenleitung auf, ist der dritte Verdichter vorzugsweise parallel zu einem Leitungsabschnitt der ersten Nebenleitung und/oder parallel zu einem Leitungsabschnitt der vierten Nebenleitung geschaltet. Der "und"-Fall ist bevorzugt. Dieser Fall kann dadurch realisiert sein, dass die erste Nebenleitung und die vierte Nebenleitung einen gemeinsamen Leitungsabschnitt aufweisen. Der dritte Verdichter ist vorzugsweise parallel zu diesem gemeinsamen Leitungsabschnitt geschaltet.

In einer weiteren bevorzugen Ausführungsform ist eine Turbine dem Auslass des Zwischenspeichers nachgeschaltet.

In dieser Ausführungsform kann die aufgebrachte Verdichtungsenergie teilweise in mechanische und/oder elektrische Energie gewandelt werden. Hierdurch kann der energetische Gesamtwirkungsgrad der Anordnung erhöht werden.

Weist die Anordnung die zweite Nebenleitung auf, ist die Turbine vorzugsweise parallel zu einem Leitungsabschnitt der zweiten Nebenleitung geschaltet. Weist die Anordnung die dritte Nebenleitung auf, ist die Turbine vorzugsweise parallel zu einem Leitungsabschnitt der dritten Nebenleitung geschaltet. Weist die Anordnung die zweite Nebenleitung und die dritte Nebenleitung auf, ist die Turbine vorzugsweise parallel zu einem Leitungsabschnitt der zweiten Nebenleitung und/oder parallel zu einem Leitungsabschnitt der dritten Nebenleitung geschaltet. Der "und"-Fall ist bevorzugt. Dieser Fall kann dadurch realisiert sein, dass die zweite Nebenleitung und die dritte Nebenleitung einen gemeinsamen Leitungsabschnitt aufweisen. Die Turbine ist vorzugsweise parallel zu diesem gemeinsamen Leitungsabschnitt geschaltet.

In einer weiteren bevorzugten Ausführungsform beinhaltet die Rohgasquelle einen Elektrolyseur. In dem Fall ist es bevorzugt, dass die Anordnung zusätzlich eine weitere Rohgasquelle für Kohlendioxid aufweist.

In einer weiteren bevorzugten Ausführungsform beinhaltet die Rohgasquelle einen Elektrolyseur, und eine weitere Rohgasquelle beinhaltet eine Kohlendioxidleitung oder eine Kohlendioxidabscheideeinheit.

In dieser Ausführungsform kann erzeugtes Methanol als zumindest teilweise nachhaltig und/oder zumindest teilweise erneuerbar bezeichnet werden. Weiterhin kann der energetische Gesamtwirkungsgrad der Methanolsynthese erhöht werden.

In einer weiteren bevorzugte Ausführungsform umfasst die Rohgasquelle eine erste Komponente und eine zweite Komponente. Die erste Komponente beinhaltet vorzugsweise Wasserstoff (H₂). Die zweite Komponente beinhaltet vorzugsweise Kohlenstoffdioxid.

Als ein weiterer Aspekt der Erfindung wird ein Verfahren zum Betrieb einer wie beschrieben ausgebildeten Anordnung vorgestellt,
wobei in einem ersten Betriebsmodus die Rohgasquelle mehr Rohgas liefert als die Synthesereaktoranordnung umsetzt, wobei überschüssiges Rohgas aus der zweiten Hauptleitung über die erste Nebenleitung dem Zwischenspeicher zugeführt und im Zwischenspeicher eingespeichert wird,
wobei in einem zweiten Betriebsmodus die Rohgasquelle weniger Rohgas liefert als die Synthesereaktoranordnung umsetzt und das Rohgas im Zwischenspeicher mit einem Druck vorliegt, welcher oberhalb eines Grenzwerts liegt, wobei Rohgas aus dem Zwischenspeicher über die zweite Nebenleitung in die zweite Hauptleitung eingespeist wird, und
wobei in einem dritten Betriebsmodus die Rohgasquelle weniger Rohgas liefert als die Synthesereaktoranordnung umsetzt und das Rohgas im Zwischenspeicher mit einem Druck vorliegt, welcher unterhalb des Grenzwerts liegt, wobei das Rohgas aus dem Zwischenspeicher über die dritte Nebenleitung in die erste Hauptleitung eingespeist wird.

Die Vorteile und Merkmale der Anordnung zur Methanolsynthese sind auf das Verfahren anwendbar und übertragbar, und umgekehrt. Die Anordnung ist vorzugsweise zum Betrieb gemäß dem beschriebenen Verfahren eingerichtet.

Die Anordnung wird je nach den vorliegenden Umständen in dem jeweils geeigneten Betriebsmodus betrieben. Es ist nicht erforderlich, dass alle drei beschriebenen Betriebsmodi durchlaufen werden. Eine feste Reihenfolge der Betriebsmodi gibt es ebenfalls nicht. Vielmehr ist es bevorzugt, dass überwacht wird, wie viel Rohgas die Rohgasquelle liefert und wie der Zustand des Zwischenspeichers ist, und dass anhand von Ergebnissen dieser Überwachung entschieden wird, in welchem Betriebsmodus die Anordnung betrieben wird. Neben den beschriebenen drei Betriebsmodi kann es weitere Betriebsmodi geben.

Der erste Betriebsmodus wird genutzt, wenn die Rohgasquelle mehr Rohgas liefert als die Synthesereaktoranordnung umsetzt. Der Zustand, dass mehr Rohgas vorhanden ist als die Synthesereaktoranordnung umsetzten kann, beruht auf unterschiedlichen Szenarien. In einem ersten Szenario wird die Synthesereaktoranordnung bei Volllast betrieben. Die Rohgasquelle produziert spontan mehr Rohgas als in der Synthesereaktoranordnung umgesetzt werden kann.

In einem zweiten Szenario wird die Synthesereaktoranordnung in Teillast betrieben. Die Rohgasquelle produziert spontan mehr Rohgas als in diesem Teillastzustand umgesetzt werden kann. Die Synthesereaktoranordnung kann dem Anstieg der Rohgasmenge aufgrund seiner Trägheit nicht folgen.

Die überschüssige Menge an Rohgas würde in beiden Szenarien ohne eine Einspeicherung im Zwischenspeicher als Abgas vor und/oder nach der Synthesereaktoranordnung ausgestoßen werden.

Der erste Betriebsmodus dient demgegenüber einer ressourcenschonenden Einspeicherung des Rohgases.

Im zweiten Betriebsmodus und im dritten Betriebsmodus liefert die Rohgasquelle weniger Rohgas als die Synthesereaktoranordnung umsetzt. Der Zustand, dass die Rohgasquelle weniger Rohgas zur Verfügung stellt als die Synthesereaktoranordnung umsetzen kann, beruht ebenfalls auf unterschiedlichen Szenarien.

In einem dritten Szenario wird die Synthesereaktoranordnung bei Volllast betrieben. Die Rohgasquelle produziert spontan weniger Rohgas als im Volllastzustand umgesetzt werden kann. Die Synthesereaktoranordnung kann dem Abfall der Rohgasmenge aufgrund ihrer Trägheit nicht ausreichend folgen.

In einem vierten Szenario wird die Synthesereaktoranordnung bei Teillast betrieben. Die Rohgasquelle produziert spontan weniger oder gar kein Rohgas als im Teillastzustand umgesetzt werden kann. Die Synthesereaktoranordnung kann dem Abfall der Rohgasmenge aufgrund ihrer Trägheit nicht ausreichend folgen. Weiterhin kann die Synthesereaktoranordnung eine minimale Teillast aufweisen, unterhalb derer die Synthesereaktoranordnung abgeschaltet wird.

Ohne eine Einspeisung des Rohgases aus dem Zwischenspeicher kann das dritte und vierte Szenario eine Einspeisung eines fossilen Rohgases zum Ausgleich des fehlenden Rohgases oder eine aufwendige Abschaltung der Synthesereaktoranordnung zur Folge haben. Im zweiten Betriebsmodus und im dritten Betriebsmodus kann dies verhindert werden, indem Rohgas aus dem Zwischenspeicher eingespeist wird.

Im zweiten Betriebsmodus liegt das Rohgas im Zwischenspeicher mit einem Druck vor, welcher oberhalb eines Grenzwerts liegt, wobei Rohgas aus dem Zwischenspeicher über die zweite Nebenleitung in die zweite Hauptleitung eingespeist wird.

Liegt das Rohgas im Zwischenspeicher mit einem Druck vor, welcher oberhalb eines Grenzwerts liegt, kann das Rohgas eingespeist werden, ohne weiter verdichtet zu werden.

Der Grenzwert kann aus dem Betriebsdruck der Synthesereaktoranordnung und dem Ausgleich von möglichen Druckverlusten zwischen dem Zwischenspeicher und der Synthesereaktoranordnung folgen.

Der zweite Betriebsmodus dient einer energieschonenden Einspeisung von Rohgas für einen sichereren Betrieb der Synthesereaktoranordnung.

Im dritten Betriebsmodus liegt das Rohgas im Zwischenspeicher mit einem Druck vor, welcher unterhalb des Grenzwerts liegt, wobei das Rohgas aus dem Zwischenspeicher über die dritte Nebenleitung in die erste Hauptleitung eingespeist wird.

Im dritten Betriebsmodus kann das Rohgas aus dem Zwischenspeicher mit dem ersten Verdichter über den Grenzwert verdichtet werden.

Der dritte Betriebsmodus dient einer energieschonenden Einspeisung von Rohgas sowie einer effizienteren Nutzung des Speichervolumens des Zwischenspeichers für einen sichereren Betrieb der Synthesereaktoranordnung.

Zur Umsetzung in der Synthesereaktoranordnung kann im zweiten und im dritten Betriebsmodus das fehlende Rohgas, welches nicht ausreichend durch die Rohgasquelle zur Verfügung gestellt wird, aus dem Zwischenspeicher entnommen werden und in Abhängigkeit vom vorliegenden Druck im Zwischenspeicher in die erste Hauptleitung oder in die zweite Hauptleitung eingespeist werden.

Weist die Anordnung die vierte Nebenleitung auf, kann die Anordnung auch in einem vierten Betriebsmodus betrieben werden. In diesem liefert die Rohgasquelle mehr Rohgas als die Synthesereaktoranordnung umsetzt, wobei überschüssiges Rohgas aus der ersten Hauptleitung über die vierte Nebenleitung dem Zwischenspeicher zugeführt und im Zwischenspeicher eingespeichert wird.

In einer bevorzugten Ausführungsform des Verfahrens beinhaltet das Rohgas der Rohgasquelle Wasserstoff, welcher zumindest teilweise durch eine Elektrolyse von Wasser mit erneuerbarer Energie erhalten wird.

Eine erste Komponente des Rohgases kann im ersten Verdichter verdichtet werden und eine zweite Komponente des Rohgases kann im parallelen zweiten Verdichter - sofern die Anordnung einen solchen aufweist - verdichtet werden. Anschließend können die erste Komponente und die zweite Komponente des Rohgases gemischt werden.

Der erste Verdichter kann zum Einspeichern von Rohgas verwendet werden. Insbesondere kann eine erste Komponente des Rohgases, bevorzugt Wasserstoff, mit dem ersten Verdichter eingespeichert werden. Eine zweite Komponente des Rohgases kann mit dem zweiten Verdichter auf einen geeigneten Betriebsdruck für die Synthesereaktoranordnung verdichtet werden. Ein paralleler zweiter Verdichter kann einen flexibleren Betrieb ermöglichen.

Sofern die Anordnung einen dritten Verdichter aufweist, kann dieser im ersten Betriebsmodus das Rohgas aus der ersten Nebenleitung und/oder der vierten Nebenleitung dem Zwischenspeicher komprimiert zuführen. Der dritte Verdichter kann das Rohgas mit einem höheren Druck im Zwischenspeicher einspeichern. Im Zwischenspeicher kann durch den höheren Druck mehr Rohgas eingespeichert werden.

Sofern die Anordnung eine Turbine aufweist, kann diese im zweiten Betriebsmodus und/oder im dritten Betriebsmodus eine Entspannungsarbeit in elektrische und/oder mechanische Arbeit wandeln. Die Turbine kann den energetischen Gesamtwirkungsgrad erhöhen.

Wärme, die in einem der Verdichter entsteht, kann genutzt werden, um Verbraucher in der Rohgasproduktion und/oder der Synthese zu heizen und/oder thermische Energie an einen externen Verbraucher zu liefern. Der energetische Gesamtwirkungsgrad kann hierdurch erhöht werden.

Die Erfindung wird nachfolgend anhand der Figuren näher erläutert. Die Figuren zeigen besonders bevorzugte Ausführungsbeispiele, auf welche die Erfindung jedoch nicht begrenzt ist. Die Figuren und die darin dargestellten Größenverhältnisse sind nur schematisch. Es zeigen:
- Fig. 1:: eine aus dem Stand der Technik bekannte Anordnung zur Methanolsyn-these in einer schematischen Darstellung,
- Fig. 2:: eine schematische Darstellung einer ersten Ausführungsform einer erfindungsgemäßen Anordnung zur Methanolsynthese,
- Fig. 3:: eine schematische Darstellung einer zweiten Ausführungsform einer erfindungsgemäßen Anordnung zur Methanolsynthese,
- Fig. 4:: eine schematische Darstellung einer dritten Ausführungsform einer erfindungsgemäßen Anordnung zur Methanolsynthese,
- Fig. 5:: eine schematische Darstellung einer vierten Ausführungsform einer erfindungsgemäßen Anordnung zur Methanolsynthese.

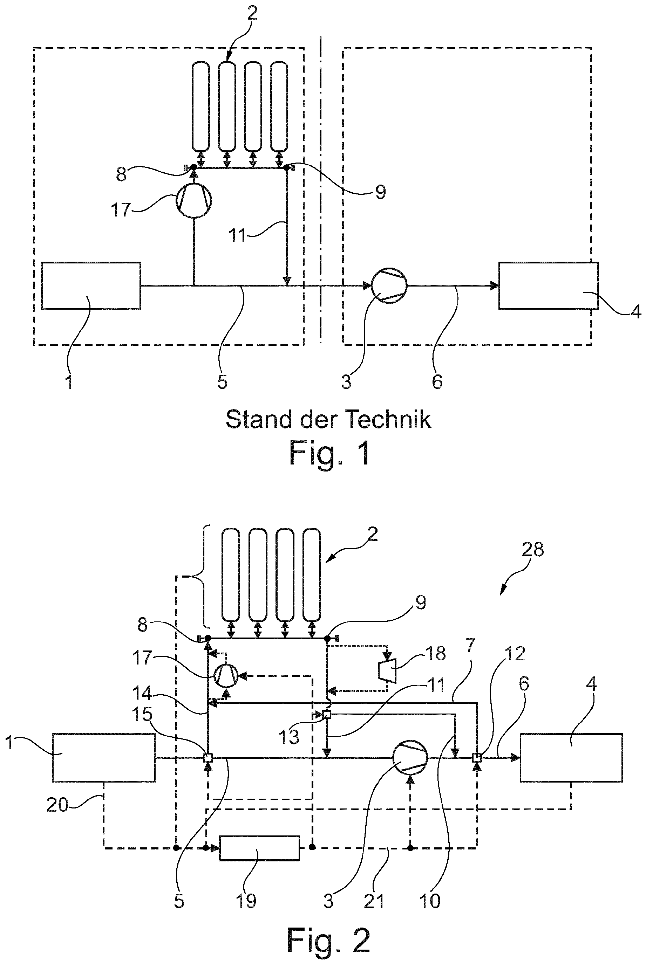

Fig. 1 zeigt eine aus dem Stand der Technik bekannte Anordnung zur Methanol-synthese. Die Anordnung umfasst eine Rohgasquelle 1, eine erste Hauptleitung, 5, eine zweite Hauptleitung 6, einen ersten Verdichter 3, einen dritten Verdichter 17, eine Synthesereaktoranordnung 4, einen Zwischenspeicher 2 und eine dritte Nebenleitung 11. Die Rohgasquelle 1 ist mit dem ersten Verdichter 3 über die erste Hauptleitung 5 verbunden. Der erste Verdichter 3 ist über die zweite Hauptleitung 6 mit der Synthesereaktoranordnung 4 verbunden. Ein Einlass 8 des Zwischenspeichers 2 ist über den dritten Verdichter 17 mit der ersten Hauptleitung 5 verbunden. Die dritte Nebenleitung 11 verbindet einen Auslass 9 des Zwischenspeichers 2 mit der ersten Hauptleitung 5. Das Rohgas wird aus der Hauptleitung 5 abgezweigt und mit dem dritten Verdichter 17 im Zwischenspeicher 2 eingespeichert. Bei Bedarf wird das Rohgas aus dem Zwischenspeicher 2 über die dritte Nebenleitung 11 mit dem ersten Verdichter 3 auf einen Betriebsdruck der Synthesereaktoranordnung 4 verdichtet. Durch eine gestrichelte Linie ist angedeutet, dass die aus dem Stand der Technik bekannte Anordnung in ein Quellen- und Speichersystem (links) und ein Synthesesystem (rechts) unterteilt ist. Dies bedingt, dass das Rohgas bei der Einspeicherung in den Zwischenspeicher 2 allein durch den dritten Verdichter 17 verdichtet werden muss. Dieser muss entsprechend ausgelegt sein, was nachteilig ist.

Fig. 2 zeigt eine schematische Darstellung einer erfindungsgemäßen Anordnung 28 zur Methanolsynthese. Die Anordnung 28 umfasst eine Rohgasquelle 1, einen Zwischenspeicher 2, einen Verdichter 3 und eine Synthesereaktoranordnung 4. Die Rohgasquelle 1 umfasst einen Elektrolyseur. Das Rohgas aus der Rohgasquelle 1 umfasst Wasserstoff, welcher teilweise durch eine Elektrolyse von Wasser mit erneuerbarer Energie erhalten wird. Das Rohgas umfasst weiterhin Kohlenstoffdioxid und/oder Kohlenstoffmonoxid. Der Zwischenspeicher 2 besteht aus mehreren Tanks und einem Rohrsystem. Die Rohgasquelle 1 ist über eine erste Hauptleitung 5 mit dem Verdichter 3 verbunden, wobei der Verdichter 3 über eine zweite Hauptleitung 6 mit der Synthesereaktoranordnung 4 verbunden ist. Die zweite Hauptleitung 6 ist über eine erste Nebenleitung 7 mit einem Einlass 8 des Zwischenspeichers 2 verbunden. Ein Auslass 9 des Zwischenspeichers 2 ist über eine zweite Nebenleitung 10 mit der zweiten Hauptleitung 6 verbunden und über eine dritte Nebenleitung 11 mit der ersten Hauptleitung 5 verbunden. Ein erstes Ventil 12 liegt zwischen der zweiten Hauptleitung 6 und der ersten Nebenleitung 7 vor. Ein zweites Ventil 13 liegt zwischen dem Auslass 9 des Zwischenspeichers 2 und der zweiten Nebenleitung 10 und der dritten Nebenleitung 11 vor. Die zweite Nebenleitung 10 ist stromaufwärts von der Verbindung der ersten Nebenleitung 7 und der zweiten Hauptleitung 6 mit der zweiten Hauptleitung 6 verbunden. Die erste Hauptleitung 5 ist über eine vierte Nebenleitung 14 mit dem Einlass 8 des Zwischenspeichers 2 verbunden. Ein drittes Ventil 15 liegt zwischen der vierten Nebenleitung 14 und der ersten Hauptleitung 5. Ein dritter Verdichter 17 ist dem Einlass 8 des Zwischenspeichers 2 vorgeschaltet. Eine Turbine 18 ist dem Auslass 9 des Zwischenspeichers 2 nachgeschaltet. Eine Steuereinheit 19 ist zur Steuerung der Ventile 12; 13; 15 und der Verdichter 3; 17 vorgesehen.

Die Anordnung 28 kann zur Methanolsynthese mit folgenden drei Betriebsmodi betrieben werden.

In einem ersten Betriebsmodus liefert die Rohgasquelle 1 mehr Rohgas als die Synthesereaktoranordnung 4 umsetzt, wobei überschüssiges Rohgas aus der zweiten Hauptleitung 6 über die erste Nebenleitung 7 dem Zwischenspeicher 2 zugeführt und im Zwischenspeicher 2 eingespeichert wird. Dabei kann das Rohgas über den dritten Verdichter 17 verdichtet werden.

In einem zweiten Betriebsmodus liefert die Rohgasquelle 1 weniger Rohgas als die Synthesereaktoranordnung 4 umsetzt. Weiterhin liegt das Rohgas im zweiten Betriebsmodus im Zwischenspeicher 2 mit einem Druck vor, welcher oberhalb eines Grenzwerts liegt, wobei Rohgas aus dem Zwischenspeicher 2 über die zweite Nebenleitung 10 in die zweite Hauptleitung 6 eingespeist wird.

In einem dritten Betriebsmodus liefert die Rohgasquelle 1 weniger Rohgas als die Synthesereaktoranordnung 4 umsetzt. Im dritten Betriebsmodus liegt das Rohgas im Zwischenspeicher 2 mit einem Druck vor, welcher unterhalb des Grenzwerts liegt, wobei das Rohgas aus dem Zwischenspeicher 2 über die dritte Nebenleitung 11 in die erste Hauptleitung 5 eingespeist wird.

Im zweiten und dritten Betriebsmodus kann die Entspannungsarbeit der Turbine 18 in elektrische Leistung gewandelt werden.

In einem vierten Betriebsmodus liefert die Rohgasquelle 1 mehr Rohgas als die Synthesereaktoranordnung 4 umsetzt, wobei überschüssiges Rohgas aus der ersten Hauptleitung 5 über die vierte Nebenleitung 14 dem Zwischenspeicher 2 zugeführt und im Zwischenspeicher 2 eingespeichert wird. Dabei kann das Rohgas über den dritten Verdichter 17 verdichtet werden.

Die Steuereinheit 19 steuert die Ventile 12;13;15 und die Verdichter 3; 17 gemäß des ersten, zweiten oder dritten Betriebsmodus. Die Steuereinheit 19 kann Eingabeparameter 20 von der Rohgasquelle 1 zur Menge des produzierten Rohgases, von dem Zwischenspeicher 2 zum Druck und Füllstand und von der Synthesereaktoranordnung 4 zur Auslastung empfangen. Die Steuereinheit 19 steuert die Ventile 12;13;15 und die Verdichter 3;17 über Ausgabeparameter 21.

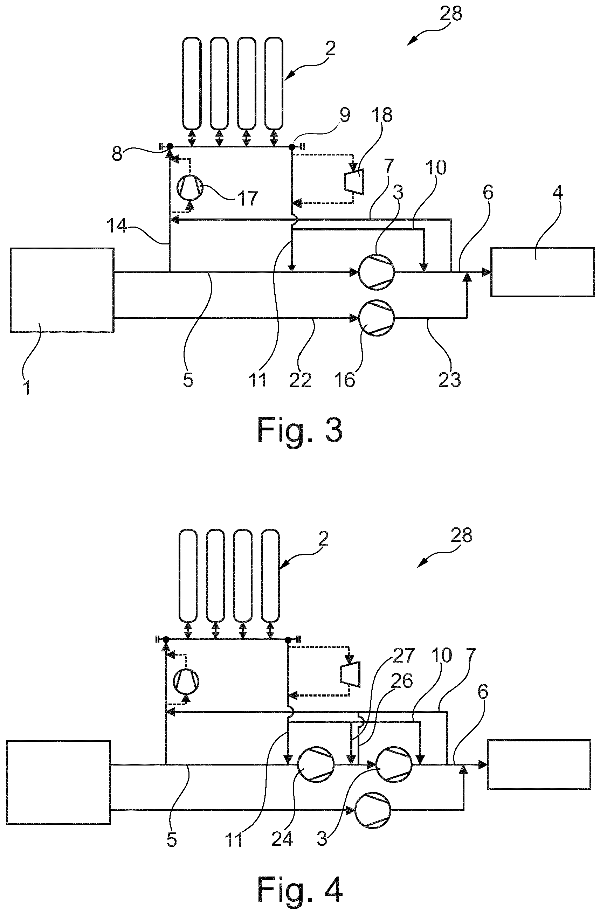

Fig. 3 zeigt eine schematische Darstellung einer zweiten Ausführungsform einer erfindungsgemäßen Anordnung 28 zur Methanolsynthese. Die Anordnung 28 umfasst über die in Fig. 2 gezeigten Komponenten einen zweiten Verdichter 16, welcher parallel zum ersten Verdichter 3 geschaltet ist. Die erste Hauptleitung 5 und die zweite Hauptleitung 6 dienen der Zuleitung einer ersten Komponente des Rohgases zu der Synthesereaktoranordnung 4. Die erste Komponente des Rohgases der Rohgasquelle 1 wird im ersten Verdichter 3 komprimiert.

Die Rohgasquelle 1 ist über eine dritte Hauptleitung 22 mit dem zweiten Verdichter 16 verbunden. Der zweite Verdichter 16 ist über eine vierte Hauptleitung 23 mit der Synthesereaktoranordnung 4 verbunden. Die dritte Hauptleitung 22 und die vierte Hauptleitung 23 dienen der Zuleitung einer zweiten Komponente des Rohgases zu der Synthesereaktoranordnung 4. Die zweite Komponente des Rohgases der Rohgasquelle 1 wird im zweiten Verdichter 16 verdichtet. Anschließend wird die zweite Komponente des Rohgases über die vierte Hauptleitung 23 in die zweite Hauptleitung 6 eingespeist.

Der erste Verdichter 3 wird zum Einspeichern der ersten Komponente von Rohgas in den Zwischenspeicher 2 verwendet. Die zweite Komponente des Rohgases wird mit dem zweiten Verdichter 16 auf einen geeigneten Betriebsdruck für die Synthesereaktoranordnung 4 verdichtet. Der parallele zweite Verdichter 16 ermöglicht einen flexibleren Betrieb.

Fig. 4 zeigt eine schematische Darstellung einer dritten Ausführungsform einer erfindungsgemäßen Anordnung 28 zur Methanolsynthese. Über die in Fig. 3 gezeigten Elemente hinaus umfasst die Anordnung 28 aus Fig. 4 weiterhin einen vierten Verdichter 24, welcher zwischen der Einspeisung der dritten Nebenleitung 11 in die erste Hauptleitung 5 und dem ersten Verdichter 3 angeordnet ist. Zwischen dem vierten Verdichter 24 und dem ersten Verdichter 3 zweigt eine fünfte Nebenleitung 26 Rohgas zur ersten Nebenleitung 7 ab und eine sechste Nebenleitung 27 speist Rohgas aus der Nebenleitung 10 ein.

In dieser Ausführungsform kann das Rohgas in unterschiedlichen Druckbereichen dem Zwischenspeicher 2 entnommen und in die erste und zweite Hauptleitung 5;6 eingespeist werden. Dies ermöglicht einen flexibleren Betrieb.

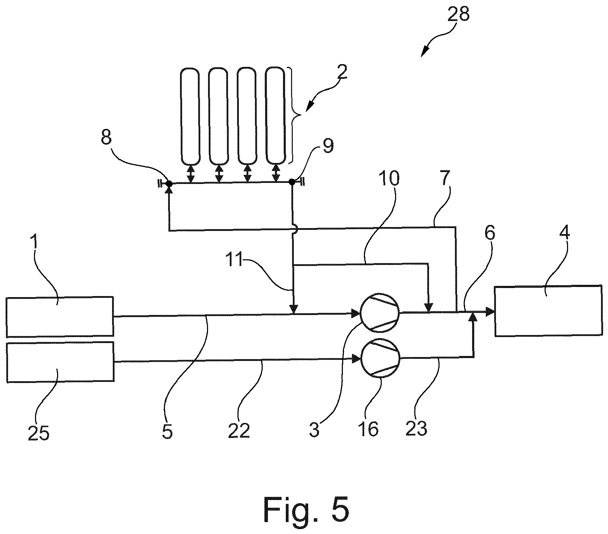

Fig. 5 zeigt eine schematische Darstellung einer vierte Ausführungsform einer erfindungsgemäßen Anordnung 28 zur Methanolsynthese. Die Anordnung 28 umfasst eine Rohgasquelle 1, eine weitere Rohgasquelle 25, einen Zwischenspeicher 2, einen ersten Verdichter 3, einen zweiten Verdichter 16, und eine Synthesereaktoranordnung 4. Die Rohgasquelle 1 für eine erste Komponente des Rohgases ist ein Elektrolyseur. Das Rohgas aus der Rohgasquelle 1 ist Wasserstoff, welcher teilweise durch eine Elektrolyse von Wasser mit erneuerbarer Energie erhalten wird. Die weitere Rohgasquelle 25 für eine zweite Komponente des Rohgases produziert Kohlenstoffdioxid aus einem Kohlenstoffdioxidabscheideprozess. Der Kohlenstoffdioxidabscheideprozess der weiteren Rohgasquelle 25 arbeitet konstant und bedarf keiner Zwischenspeicherung im Zwischenspeicher 2.

Der Zwischenspeicher 2 besteht aus mehreren Tanks und einem Rohrsystem. Die Rohgasquelle 1 ist über eine erste Hauptleitung 5 mit dem Verdichter 3 verbunden, wobei der Verdichter 3 über eine zweite Hauptleitung 6 mit der Synthesereaktoranordnung 4 verbunden ist.

Die weitere Rohgasquelle 25 ist über eine dritte Hauptleitung 22 mit dem zweiten Verdichter 16 verbunden. Der zweite Verdichter 16 ist über eine vierte Hauptleitung 23 mit der zweiten Hauptleitung 6 verbunden. Insoweit ist der zweite Verdichter 16 über die vierte Hauptleitung 23 mit der Synthesereaktoranordnung 4 verbunden.

Die zweite Hauptleitung 6 ist über eine erste Nebenleitung 7 mit einem Einlass 8 des Zwischenspeichers 2 verbunden. Ein Auslass 9 des Zwischenspeichers 2 ist über eine zweite Nebenleitung 10 mit der zweiten Hauptleitung 6 verbunden und über eine dritte Nebenleitung 11 mit der ersten Hauptleitung 5 verbunden. Die zweite Nebenleitung 10 ist stromaufwärts von der Verbindung der ersten Nebenleitung 7 und der zweiten Hauptleitung 6 mit der zweiten Hauptleitung 6 verbunden. Die Anordnung 28 kann zur Methanolsynthese mit folgenden drei Betriebsmodi betrieben werden.

In einem ersten Betriebsmodus liefert die Rohgasquelle 1 mehr Rohgas als die Synthesereaktoranordnung 4 umsetzt, wobei überschüssiges Rohgas aus der zweiten Hauptleitung 6 über die erste Nebenleitung 7 dem Zwischenspeicher 2 zugeführt und im Zwischenspeicher 2 eingespeichert wird.

In einem zweiten Betriebsmodus liefert die Rohgasquelle 1 weniger Rohgas als die Synthesereaktoranordnung 4 umsetzt. Weiterhin liegt das Rohgas im zweiten Betriebsmodus im Zwischenspeicher 2 mit einem Druck vor, welcher oberhalb eines Grenzwerts liegt, wobei Rohgas aus dem Zwischenspeicher 2 über die zweite Nebenleitung 10 in die zweite Hauptleitung 6 eingespeist wird.

In einem dritten Betriebsmodus liefert die Rohgasquelle 1 weniger Rohgas als die Synthesereaktoranordnung 4 umsetzt. Im dritten Betriebsmodus liegt das Rohgas im Zwischenspeicher 2 mit einem Druck vor, welcher unterhalb des Grenzwerts liegt, wobei das Rohgas aus dem Zwischenspeicher 2 über die dritte Nebenleitung 11 in die erste Hauptleitung 5 eingespeist wird.

Während des ersten, zweiten und dritten Betriebsmodus liefert die Rohgasquelle 25 konstant die zweite Komponente des Rohgases.

### Bezugszeichen

- 1: Rohgasquelle
- 2: Zwischenspeicher
- 3: erster Verdichter
- 4: Synthesereaktoranordnung
- 5: erste Hauptleitung
- 6: zweite Hauptleitung
- 7: erste Nebenleitung
- 8: Einlass
- 9: Auslass
- 10: zweite Nebenleitung
- 11: dritte Nebenleitung
- 12: erstes Ventil
- 13: zweites Ventil
- 14: vierte Nebenleitung
- 15: drittes Ventil
- 16: zweiter Verdichter
- 17: dritter Verdichter
- 18: Turbine
- 19: Steuereinheit
- 20: Eingabeparameter
- 21: Ausgabeparameter
- 22: dritte Hauptleitung
- 23: vierte Hauptleitung
- 24: vierter Verdichter
- 25: zweite Rohgasquelle
- 26: fünfte Nebenleitung
- 27: sechste Nebenleitung
- 28: Anordnung

## Patentansprüche

1. Anordnung (28) zur Methanolsynthese umfassend:
▪ eine Rohgasquelle (1),
▪ einen Zwischenspeicher (2),
▪ einen Verdichter (3),
▪ eine Synthesereaktoranordnung(4),
wobei die Rohgasquelle (1) über eine erste Hauptleitung (5) mit dem Verdichter (3) verbunden ist, wobei der Verdichter (3) über eine zweite Hauptleitung (6) mit der Synthesereaktoranordnung(4) verbunden ist,
wobei die zweite Hauptleitung (6) über eine erste Nebenleitung (7) mit einem Einlass (8) des Zwischenspeichers (2) verbunden ist,
wobei ein Auslass (9) des Zwischenspeichers (2) über eine zweite Nebenleitung (10) mit der zweiten Hauptleitung (6) verbunden ist und/oder über eine dritte Nebenleitung (11) mit der ersten Hauptleitung (5) verbunden ist.

2. Anordnung (28) nach Anspruch 1, wobei die erste Hauptleitung (5) über eine vierte Nebenleitung (14) mit dem Einlass (8) des Zwischenspeichers (2) verbunden ist.

3. Anordnung (28) nach einem der vorherigen Ansprüche, weiterhin umfassend einen zweiten Verdichter (16), welcher parallel zum ersten Verdichter (3) geschaltet ist.

4. Anordnung (28) nach einem der vorherigen Ansprüche, wobei ein dritter Verdichter (17) dem Einlass (8) des Zwischenspeichers (2) vorgeschaltet ist.

5. Anordnung (28) nach einem der vorherigen Ansprüche, wobei eine Turbine (18) dem Auslass (9) des Zwischenspeichers (2) nachgeschaltet ist.

6. Anordnung (28) nach einem der vorherigen Ansprüche, wobei die Rohgasquelle (1) einen Elektrolyseur beinhaltet

7. Verfahren zum Betrieb einer Anordnung (28) nach einem der Ansprüche 1 bis 6, wobei in einem ersten Betriebsmodus die Rohgasquelle (1) mehr Rohgas liefert als die Synthesereaktoranordnung (4) umsetzt, wobei überschüssiges Rohgas aus der zweiten Hauptleitung (6) über die erste Nebenleitung (7) dem Zwischenspeicher (2) zugeführt und im Zwischenspeicher (2) eingespeichert wird,
wobei in einem zweiten Betriebsmodus die Rohgasquelle (1) weniger Rohgas liefert als die Synthesereaktoranordnung (4) umsetzt und das Rohgas im Zwischenspeicher (2) mit einem Druck vorliegt, welcher oberhalb eines Grenzwerts liegt, wobei Rohgas aus dem Zwischenspeicher (2) über die zweite Nebenleitung (10) in die zweite Hauptleitung (6) eingespeist wird, und
wobei in einem dritten Betriebsmodus die Rohgasquelle (1) weniger Rohgas liefert als die Synthesereaktoranordnung (4) umsetzt und das Rohgas im Zwischenspeicher (2) mit einem Druck vorliegt, welcher unterhalb des Grenzwerts liegt, wobei das Rohgas aus dem Zwischenspeicher (2) über die dritte Nebenleitung (11) in die erste Hauptleitung (5) eingespeist wird.

8. Verfahren nach Anspruch 7, wobei das Rohgas aus der Rohgasquelle (1) Wasserstoff beinhaltet, welcher zumindest teilweise durch eine Elektrolyse von Wasser mit erneuerbarer Energie erhalten wird.
